# EUROPEAN PATENT APPLICATION

(11) **EP 4 759 249 A1**
(43) Date of publication of application: **17.06.2026**
(21) Application number: 24853602.1
(22) Date of filing: 06.08.2024
(51) Int. Cl.: A61B 17/3203

(54) **MEDICAL WATER JET SCALPEL INSTRUMENT AND MEDICAL WATER JET SCALPEL SYSTEM**

(30) Priority: 11.08.2023 CN 202311014174
(71) Applicant: Bluesail Surgical Co., Ltd., Shanghai 201321 (CN)
(72) Inventor: DING, Weijiang, Shanghai 201321 (CN); YANG, Shenghua, Shanghai 201321 (CN)
(74) Representative: Viering, Jentschura & Partner mbB Patent- und Rechtsanwälte
(86) International application number: PCT/CN2024/109947
(87) International publication number: WO 2025/036191

(57) **Abstract**

The present disclosure provides a medical water jet scalpel instrument and a medical water jet scalpel system. The medical water jet scalpel instrument comprises an outer tube, a spray tube, and a driving device. The outer tube comprises a first tube body extending in a first direction and a first distal end portion having a first distal-side tube opening end surface, and the first distal-side tube opening end surface is arranged to be inclined to the first direction. The spray tube extends through the outer tube; a medical liquid is input from a proximal end of the spray tube; a gap is formed between the outer wall of the spray tube and the inner wall of the outer tube; a spray hole is formed in a distal end of the spray tube; and the medical liquid is sprayed in a second direction inclined to the first direction. The driving device is connected to at least part of the spray tube, and drives the spray tube to move. According to the present disclosure, by inclining the axis of the outer tube body to the tube opening of the outer tube, and ingeniously designing the outer tube and/or an inner tube, the medical liquid is sprayed in another direction inclined to the direction of the outer tube body, such that in, for example, open surgery or endoscopic surgery, the operation of an operator can be more flexible, the clinical functional requirements are met, and great advantages in matching performance, processability and assemblability are achieved.

## Description

For all purposes, the present application claims priority to Chinese Patent Application No. 202311014174.9 filed on August 11, 2023, the entire content of which is incorporated herein by reference as part of the present application.

### TECHNICAL FIELD

The present disclosure relates to the field of medical devices, and in particular to a medical water jet device and a medical water jet system.

### BACKGROUND

Treatment methods for liver cancer include surgical treatment, local ablation, transcatheter arterial chemoembolization, radiotherapy, and systemic therapy. Currently, surgical treatments such as a medical water jet device and a surgical scalpel remain the preferred methods for liver cancer treatment.

The liver is an organ with a very complex structure and abundant blood supply. The liver mainly includes two different types of tissues: one is hepatic parenchyma, and the other is luminal tissue. The hepatic parenchyma is soft, while the luminal tissue is tough. These two types of tissues have significant differences in mechanical strength. Under the impact of the jet flow from the medical water jet device, the hepatic parenchyma breaks and fragments to be removed, while the luminal tissue remains intact and is preserved. Thus, the medical water jet device can roughly achieve a selective separation function.

However, as the field of medical surgery is developing towards minimally invasive and precise-oriented directions, none of the numerous liver resection methods can fully satisfy various surgical needs for liver resection, effectively control postoperative complications, and improve 5-year postoperative survival rates. Therefore, further innovation of existing medical technologies is imperative.

### SUMMARY

At least one embodiment of the present disclosure provides a medical water jet device, including: an outer tube, including a first tube body extending along a first direction and a first distal portion having a first distal outlet end face, where a plane defined by the first distal outlet end face is obliquely arranged relative to the first direction; a nozzle tube, extending through the outer tube and configured to input medical liquid at a proximal end, where a gap space is provided between an outer wall of the nozzle tube and an inner wall of the outer tube, an orifice configured to output the medical liquid is provided at a distal end of the nozzle tube, and the medical liquid is ejected along a second direction that is oblique to the first direction; and a driving device, connected to at least a portion of the nozzle tube and configured to drive the nozzle tube to move.

For example, in the medical water jet device provided by at least one embodiment of the present disclosure, the first distal portion of the outer tube is in a curved shape; and the nozzle tube includes a second tube body extending along the first direction and a second distal portion in a curved shape.

For example, in the medical water jet device provided by at least one embodiment of the present disclosure, the first distal outlet end face is perpendicular to a central axis of the first distal portion, and a second distal outlet end face on the second distal portion is perpendicular to a central axis of the second distal portion.

For example, in the medical water jet device provided by at least one embodiment of the present disclosure, a central axis of the first distal portion is parallel to a central axis of the second distal portion.

For example, in the medical water jet device provided by at least one embodiment of the present disclosure, the first tube body of the outer tube is continuous and coaxial with the first distal portion, and the first distal outlet end face of the outer tube is obliquely arranged relative to the first direction; and the nozzle tube is arranged to extend along the first direction, and a second distal outlet end face of the nozzle tube is obliquely arranged relative to the first direction.

For example, in the medical water jet device provided by at least one embodiment of the present disclosure, the plane defined by the first distal outlet end face is parallel to a plane defined by the second distal outlet end face.

For example, in the medical water jet device provided by at least one embodiment of the present disclosure, the first tube body of the outer tube is continuous and coaxial with the first distal portion, and the first distal outlet end face of the outer tube is obliquely arranged relative to the first direction; and the nozzle tube is arranged to extend along the first direction, a second distal outlet end face of the nozzle tube is perpendicular to the first direction, and the orifice is configured in an oblique orifice shape relative to the second distal outlet end face so that the medical liquid is ejected along the second direction.

For example, in the medical water jet device provided by at least one embodiment of the present disclosure, the second direction is perpendicular to the first distal outlet end face.

For example, in the medical water jet device provided by at least one embodiment of the present disclosure, an acute angle formed between the second direction and the first direction is 10° to 50°.

For example, in the medical water jet device provided by at least one embodiment of the present disclosure, a proximal portion of the nozzle tube is configured to be fixed relative to the outer tube, and the driving device is configured to drive the nozzle tube such that the orifice of the nozzle tube performs, relative to the outer tube, a one-dimensional reciprocating swing along a radial direction of the outer tube or a two-dimensional circular rotation around a direction of a central axis of the outer tube.

At least one embodiment of the present disclosure further provides a medical water jet system, including a liquid supply unit, a pressure pump, and the medical water jet device as described in any one of the above. The liquid supply unit is configured to provide the medical liquid. The pressure pump is configured to apply a pressure to the medical liquid to input the medical liquid into the nozzle tube.

Compared with the prior art, the beneficial effects of at least one embodiment of the present disclosure include at least: by arranging the outlet of the outer tube to be oblique to but not perpendicular to the axis line of the main body of the outer tube, and cleverly designing the outer tube and/or the inner tube to enable the jet flow direction to form an oblique angle with relative to the central axis of the main body of the outer tube, the medical water jet device of the present disclosure makes the operation of the operator more flexible, both in open surgical procedures and endoscopic surgical procedures, which not only satisfies clinical functional requirements but also achieves technical effects with great advantages in terms of compatibility, machinability, and assemblability.

### BRIEF DESCRIPTION OF DRAWINGS

To more clearly illustrate the embodiments of the present disclosure or the technical solutions in the prior art, the drawings needed in the description of the embodiments or the prior art will be briefly introduced below. Obviously, the drawings in the following description are only some embodiments of the present disclosure. For those ordinary skilled in the art, other drawings can be obtained according to these drawings without creative work.
Fig. 1 is a schematic cross-sectional view of a medical water jet device provided by some embodiments of the present disclosure;
Fig. 2 is a schematic diagram of working principle of one-dimensional reciprocating swing of a water jet nozzle of a medical water jet device provided by some embodiments of the present disclosure;
Fig. 3 is a schematic diagram of working principle of two-dimensional circular rotation of a water jet nozzle of a medical water jet device provided by some embodiments of the present disclosure;

Figs. 4-5 are schematic structural diagrams of an outer tube and a nozzle tube both provided with bent outlets according to some embodiments of the present disclosure, wherein Fig. 5 is a left view of Fig. 4;

Figs. 6-7 are schematic structural diagrams of an outer tube and a nozzle tube both provided with beveled outlets according to some embodiments of the present disclosure, wherein Fig. 7 is a left view of Fig. 6; and

Figs. 8-9 are schematic structural diagrams of an outer tube provided with a beveled outlet and a nozzle tube provided with an oblique orifice according to some embodiments of the present disclosure, wherein Fig. 9 is a left view of Fig. 8.

In drawings: 100-medical water jet device, 110-outer tube, 111-first tube body, 112-first distal portion, 112a-first distal outlet end face, 120-nozzle tube, 121-second tube body, 122-second distal portion, 122a-second distal outlet end face, 130-driving device.

### DETAILED DESCRIPTION

The technical solutions in the embodiments of the present disclosure can be clearly and completely described below in conjunction with the drawings in the embodiments of the present disclosure. Obviously, the described embodiments are only a part of the embodiments of the present disclosure, not all of them. Based on the embodiments in the present disclosure, all other embodiments obtained by those ordinary skilled in the art without creative work shall fall within the scope of protection of the present disclosure.

Unless otherwise defined, all terms (including technical and scientific terms) used in the embodiments of the present disclosure have the same meanings as commonly understood by those ordinary skilled in the art to which the present disclosure belongs. It should also be understood that terms such as those defined in commonly used dictionaries should be interpreted as having meanings consistent with their meanings in the context of related technologies, and shall not be interpreted in an idealized or overly formal sense unless explicitly defined as such in the embodiments of the present disclosure.

The terms such as "first" and "second" used in the embodiments of the present disclosure do not indicate any order, quantity, or importance, but are only used to distinguish different components. The terms such as "a", "an" or "the" do not indicate a quantity limitation, but indicate the presence of at least one. Similarly, the terms such as "comprising" or "including" mean that the element or item preceding the terms includes the element or item listed after the terms and its equivalents, but does not exclude other elements or items. The terms such as "connecting" or "connected" are not limited to physical or mechanical connections, but may include electrical connections or communication connections, whether direct or indirect. Flowcharts are used in the embodiments of the present disclosure to illustrate the steps of the method according to the embodiments of the present disclosure. It should be understood that the previous or subsequent steps are not necessarily performed in exact order. Instead, various steps can be processed in reverse order or simultaneously unless explicitly limited by the embodiments of the present disclosure. At the same time, other operations can be added to these processes, or a step or steps can be removed from these processes.

In the surgical treatment of the liver, an ideal liver resection method should have the following characteristics: fast speed, no thermal injury, ability to protect lumens, realization of precise separation, minimal blood loss, easy operation under laparoscopy, low risk of bile leakage and effusion, low cost, and an ideal surgical environment.

The technology of medical water jet devices has great potential when applied in the fields of liver resection. For example, during operation, the medical water jet device can complete the cutting or removal of hepatic parenchyma and the preservation and exposure of luminal tissue in a cutting zone with a certain width and length. This method causes no thermal injury to luminal tissue and parenchymal tissue. When the luminal tissue is clearly visible, the closure and transection operations can also protect the luminal tissue and reduce surgical risks. During the process, blood loss is minimal, and bile leakage and effusion are not easy to occur. However, in most medical water jet devices, the axis line of the handle, the axis line of the outer tube, and the jet flow direction of the medical liquid are parallel to each other, which leads to limited direction during surgical operation, thus causing great difficulties for operators (such as surgeons) in use, especially under laparoscopy.

If a water jet nozzle of the medical water jet device rotates on its own axis (i.e., the orientation of the water jet nozzle itself deflects when the nozzle tube rotates on its own axis, where the rotation of the nozzle tube on its own axis refers to the rotation of the nozzle tube around its central axis), it can easily cause the deflection direction of the jet flow to change periodically, which makes it difficult to satisfy the operator's requirements.

At least one embodiment of the present disclosure provides a medical water jet device, including an outer tube, a nozzle tube, and a driving device. The outer tube includes a first tube body extending along a first direction and a first distal portion having a first distal outlet end face obliquely arranged relative to the first direction. The nozzle tube extends through the outer tube and is configured to input medical liquid at a proximal end. A gap space is provided between an outer wall of the nozzle tube and an inner wall of the outer tube. An orifice that is configured to output the medical liquid is provided at a distal end of the nozzle tube, and the medical liquid is ejected along a second direction that is oblique to the first direction. The driving device is connected to at least a portion of the nozzle tube and configured to drive the nozzle tube to move.

In the medical water jet device of the above embodiment of the present disclosure, the outlet of the outer tube is designed to be obliquely arranged relative to the axis line of the main body of the outer tube; i.e., the plane where the outlet of the outer tube is located intersects with the axis line of the main body of the outer tube, but is not perpendicular thereto. This enables the jet flow direction to form an oblique angle relative to the central axis of at least a portion of the outer tube. Therefore, such design with oblique angle makes the operation of the operator more flexible, both in open surgical procedures and endoscopic surgical procedures, which not only satisfies clinical functional requirements but also achieves technical effects with great advantages in terms of compatibility, machinability, and assemblability.

The structural solution proposed in at least one embodiment of the present disclosure, first of all, satisfies clinical functional requirements for the jet flow direction to deflect by a certain angle relative to the axial direction of the outer tube; secondly, it satisfies structural movement requirements of the system for the device in the working state; in addition, it can achieve better compatibility with a 5 mm trocar, as well as superior machinability and assemblability, thus yielding greater economic benefits.

The embodiments of the present disclosure and their examples can be described in detail below with reference to the drawings.

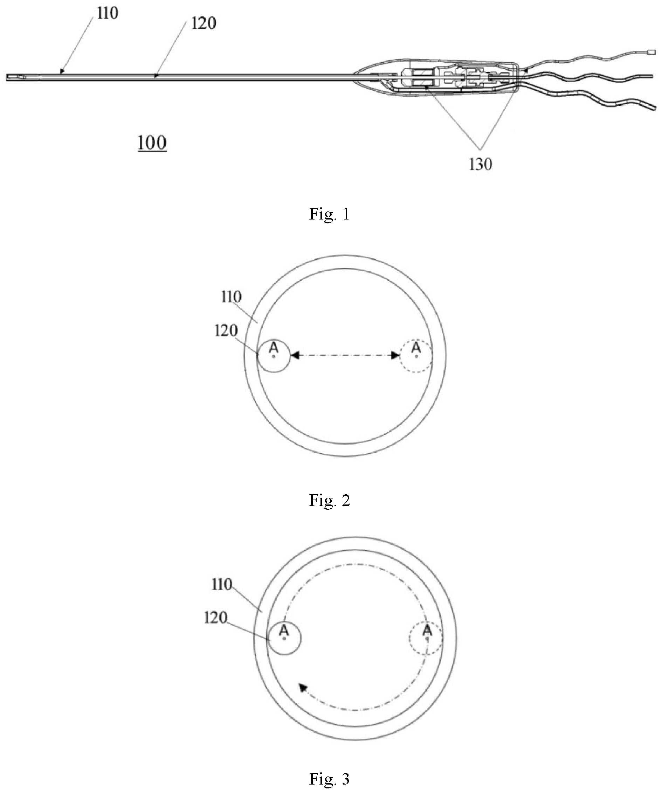

For the convenience of description, the proximal end of the outer tube 110 of the medical water jet device 100 in Fig. 1 of the embodiment of the present disclosure refers to the side closer to the operator, and the distal end of the outer tube 110 is the side away from the operator. In at least one embodiment of the present disclosure, the side closer to the operator (such as a surgeon) is regarded as the proximal end or proximal side, and the side away from the operator is regarded as the distal end or distal side. It should be noted that the distal end, proximal end, etc., in the embodiments of the present disclosure are all relative positions. For example, they represent two opposite sides of some components themselves, or two opposite sides in a certain direction, i.e., the proximal end in the embodiments of the present disclosure represents one side, and the distal end represents the other side opposite to the proximal end.

For the clarity and conciseness of the present disclosure, the following description mainly refers to the case where the flexible tissue is the hepatic parenchymal tissue and the elastic tissue is the luminal tissue, by way of examples. However, the embodiments of the present disclosure impose no limitation on the types of flexible tissue and elastic tissue to which the present disclosure are applicable, i.e., the present disclosure can also be applied to other biological tissues with similar needs and characteristics, such as parenchymal tissues and luminal tissues (such as blood vessels, bile ducts, ureters, bronchi, etc.) corresponding to other human organs (such as lungs, kidneys, spleens, etc.), which can be determined according to actual conditions and are not limited in the embodiments of the present disclosure.

Fig. 1 is a schematic cross-sectional view of a medical water jet device provided by some embodiments of the present disclosure.

For example, as shown in Fig. 1, a medical water jet device 100 provided by at least one embodiment of the present disclosure includes an outer tube 110, a nozzle tube 120, and a driving device 130. The nozzle tube 120 extends through the outer tube 110 and is configured to input a pressurized medical liquid and allow the medical liquid to circulate. A gap space is provided between the outer wall of the nozzle tube 120 and the inner wall of the outer tube 110 (i.e., the space formed by the inner wall of the outer tube 110 and the outer wall of the nozzle tube 120). The nozzle tube 120 may also be referred to as an inner tube. The driving device 130 is connected to at least a portion of the nozzle tube 120 and drives the nozzle tube 120 to move. For example, the driving device 130 drives the distal end of the nozzle tube 120 to perform, relative to the outer tube 110, a one-dimensional reciprocating swing along the radial direction of the outer tube 110, or a two-dimensional circular rotation around the central axis of the outer tube 110. As the nozzle tube 120 moves, the gap space between the outer wall of the nozzle tube 120 and the inner wall of the outer tube 110 changes.

For example, the distal end of the nozzle tube 120 is provided with an orifice (not shown in Fig. 1) configured to output the medical liquid so that the medical liquid is ejected in a certain direction (such as the second direction described below), thereby realizing the selective separation of, for example, hepatic parenchyma from blood vessels and bile ducts in the body by using the jet flow. For example, the jet flow generated in the embodiments of the present disclosure can efficiently complete the selective separation of flexible tissues and elastic tissues.

In some examples, the orifice may be directly opened in the distal end of the nozzle tube 120 (i.e., it can also be understood as the distal portion of the nozzle tube 120 including a water jet nozzle and the water jet nozzle includes the orifice). The orifice may also be opened in a water jet nozzle (not shown in Fig. 1) fixedly installed at the distal end of the nozzle tube 120, and the water jet nozzle is fixed at the distal end of the nozzle tube 120 as a separate component which is independent of the nozzle tube 120.

In some examples, the driving device 130 drives, by driving a movement of the nozzle tube 120, the water jet nozzle at the distal end of the nozzle tube 120 to move, and the movement of the water jet nozzle is consistent with the movement of the distal end of the nozzle tube 120. For example, the proximal end of the nozzle tube 120 moves (e.g., swings) under the driving of the driving device 130, and this movement is transmitted to the distal end of the nozzle tube 120 and the water jet nozzle at the distal end, so that the water jet nozzle performs corresponding movement. Therefore, the moving water jet nozzle enables the high-pressure jet flow ejected from its orifice to realize an automatic scanning action.

It should be noted that since the specific design of the water jet nozzle and the orifice is not the focus of the description of the embodiments of the present disclosure, it is not particularly explained here. For the clarity and conciseness of the present disclosure, the following description mainly refers to the case where the orifice is directly opened in the distal end of the nozzle tube 120 by way of example, but the embodiments of the present disclosure are not limited thereto, and is not exhaustively described here.

For example, the inner diameter of the outer tube 110 of the medical water jet device 100 is larger than the outer diameter of the nozzle tube 120 of the medical water jet device 100. For example, the medical liquid may include physiological saline. For example, the medical water jet device 100 is a disposable surgical instrument that can be operated with a single hand by a surgeon. For example, the medical water jet device 100 may further include a handle, the driving device 130 is arranged in the handle, and the handle is sleeved on the outer side of the proximal end of the outer tube 110 and fixedly connected to the proximal end of the outer tube 110. It should be noted that these are only exemplary and not limitations of the present disclosure. For example, in some other embodiments, the driving device 130 may also be located outside the handle, which is not the focus of the description of the embodiments of the present disclosure and is not particularly explained here.

Fig. 2 is a schematic diagram of working principle of a one-dimensional reciprocating swing of a water jet nozzle of a medical water jet device provided by some embodiments of the present disclosure. Fig. 3 is a schematic diagram of working principle of a two-dimensional circular rotation of a water jet nozzle of a medical water jet device provided by some embodiments of the present disclosure.

For example, as shown in Figs. 2 and 3, the nozzle tube 120 is disposed inside the outer tube 110. The small solid-line circle at the left and the small dashed-line circle at the right, which are inside the outer tube 110, represent the possible positions of the water jet nozzle (and the orifice) positioned at the distal end of the nozzle tube 120 at two movement moments, respectively. The dash-dot lines and arrows represent the scanning path and the movement direction of the water jet nozzle, and the character "A" can be used to represent the orientation of the water jet nozzle itself, i.e., if "A" is inverted, it means that the water jet nozzle has been rotated by 180° at the corresponding position.

For example, the example in Fig. 2 corresponds to the case where the distal end of the nozzle tube 120 performs, relative to the outer tube 110, a one-dimensional reciprocating swing along the radial direction of the outer tube 110 (which may also be described as "the nozzle tube 120 performs lateral swing inside the outer tube 110). For example, the example shown in Fig. 3 corresponds to the case where the distal end of the nozzle tube 120 performs a two-dimensional circular rotation around the central axis of the outer tube 110 (which may also be described as "the nozzle tube 120 performs orbital rotation inside the outer tube 110"). It can be seen from Figs. 2 and 3 that when the distal end of the nozzle tube 120 moves, regardless of the path, the orientation of the water jet nozzle itself does not deflect, i.e., the water jet nozzle does not rotate on its own axis.

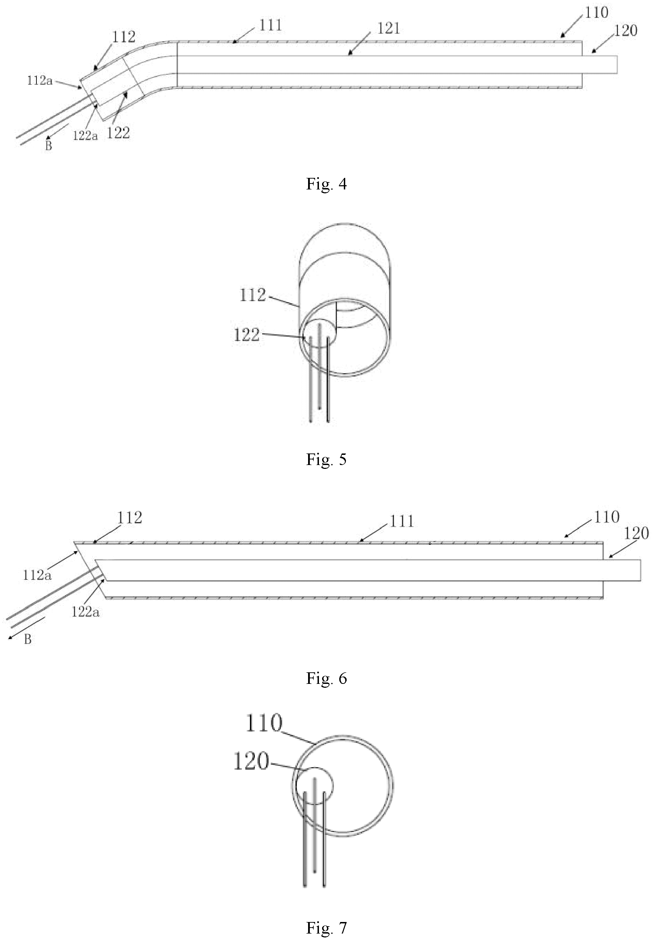

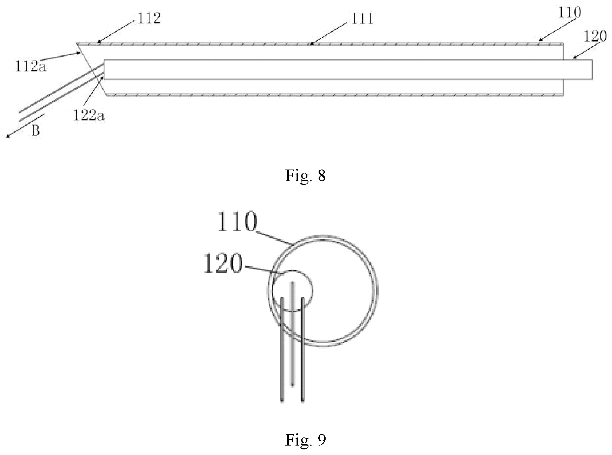

Figs. 4-5 are schematic structural diagrams of an outer tube and a nozzle tube both provided with bent outlets according to some embodiments of the present disclosure, and Fig. 5 is a left view of Fig. 4. Figs. 6-7 are schematic structural diagrams of an outer tube and a nozzle tube both provided with beveled outlets according to some embodiments of the present disclosure, and Fig. 7 is a left view of Fig. 6. Figs. 8-9 are schematic structural diagrams of an outer tube provided with a beveled outlet and a nozzle tube provided with an oblique orifice according to some embodiments of the present disclosure, and Fig. 9 is a left view of Fig. 8.

For example, as shown in Figs. 4, 6, and 8, the outer tube 110 includes a first tube body 111 extending along a first direction and a first distal portion 112 having a first distal outlet end face 112a, and a plane where the first distal outlet end face 112a is located is obliquely arranged relative to the first direction. For example, the first direction corresponds to the left-right direction on the drawings in Figs. 1, 4, 6, and 8. For example, the distal end of the nozzle tube 120 is provided with an orifice (not shown) configured to output medical liquid, and the medical liquid is ejected along a second direction that is oblique to the first direction. For example, the second direction corresponds to the direction indicated by the arrow B on the drawings in Figs. 4, 6, and 8.

In the medical water jet device of the above embodiment of the present disclosure, the outlet of the outer tube is designed to be obliquely arranged relative to the axis line of the main body of the outer tube, so that the jet flow direction can form an oblique angle with relative to the central axis of at least a portion of the outer tube, which is beneficial to expanding the operation range of the medical water jet device during surgery, enabling the use of the device to be more flexible, and satisfying the clinical functional requirements of users for the device.

In the embodiments of the present disclosure, that at least a portion of the outer tube 110 or at least a portion of the nozzle tube 120 extends along the first direction means that the central axis of this portion coincides with the first direction or is parallel to the first direction.

In some examples, a surface of the tissue to be treated is configured to fit the first distal outlet end face 112a of the outer tube 110. This is only exemplary and does not constitute a limitation of the embodiments of the present disclosure.

For example, as shown in Figs. 4 and 5, the first distal portion 112 of the outer tube 110 is in a curved shape, i.e., the first distal portion 112 of the outer tube 110 is not coaxial with the first tube body 111 of the outer tube 110.

For example, as shown in Figs. 4 and 5, the nozzle tube 120 includes a second tube body 121 extending along the first direction and a second distal portion 122 in a curved shape, i.e., the second distal portion 122 of the nozzle tube 120 is not coaxial with the second tube body 121 of the nozzle tube 120.

In some examples, both the outer tube 110 and the nozzle tube 120 are straight tubes and have good cylindricity (e.g., when the intended state is achieved). For example, in some embodiments of the present disclosure, the first tube body 111 and the second tube body 121 are maintained in a nearly straight tube state during surgery by designing reasonable lengths and diameters of the outer tube 110 and the nozzle tube 120, as well as the materials of the tube bodies. For example, the first distal portion 112 in a curved shape and the second distal portion 122 in a curved shape in some embodiments of the present disclosure refer to bent portions in a stable state after being shaped and bent. Thus, for the outer tube 110 and the nozzle tube 120, they are not entirely bent during surgery but only partially bent at the distal ends.

For example, as shown in Fig. 4, the first distal outlet end face 112a of the outer tube 110 is perpendicular to the central axis of the first distal portion 112, i.e., the first distal outlet end face 112a of the outer tube 110 is in a straight outlet shape (also referred to as a flat outlet shape) perpendicular to the central axis of the first distal portion 112. The second distal outlet end face 122a on the second distal portion 122 is perpendicular to the central axis of the second distal portion 122, i.e., the second distal outlet end face 122a on the second distal portion 122 is in a straight outlet shape (also referred to as a flat outlet shape) perpendicular to the central axis of the second distal portion 122.

For example, in the example of Fig. 4, the second direction corresponding to the jet flow direction is perpendicular to the first distal outlet end face 112a of the outer tube 110.

Due to the features of the two bent outlets at the distal end of the outer tube and the distal end of the nozzle tube, the embodiments of the present disclosure enable the jet flow direction to deflect by a certain angle relative to the axial direction of the main body of the outer tube, which can satisfy clinical functional requirements.

For example, as shown in Fig. 4, the central axis of the first distal portion 112 of the outer tube 110 is parallel to the central axis of the second distal portion 122 of the nozzle tube 120, i.e., the curvature of the bent portion of the first distal portion 112 of the outer tube 110 is the same as that of the bent portion of the second distal portion 122 of the nozzle tube 120, with the same bending directions. Exemplarily, the central axis of the first distal portion 112 and the central axis of the second distal portion 122 are both curved axes, and the two curved axes are arranged in parallel to each other, i.e., the distance between corresponding positions in the two curved axes is equal. In this way, the embodiments of the present disclosure can not only satisfy clinical functional requirements but also enhance the advantages in terms of compatibility, machinability, and assemblability.

For example, as shown in Figs. 6 and 7, the first tube body 111 of the outer tube 110 is continuous and coaxial with the first distal portion 112 of the outer tube 110 (i.e., the outer tube 110 extends along the first direction as a whole, no bent outlet is provided at the distal end of the outer tube 110, and the central axis of the entire outer tube 110 is along the first direction). The first distal outlet end face 112a of the outer tube 110 is obliquely arranged relative to the first direction (the first direction intersects with the first distal outlet end face 112a but is not perpendicular thereto), i.e., the first distal outlet end face 112a of the outer tube 110 is in a beveled outlet shape relative to the first direction.

In some examples, the first tube body 111 of the outer tube 110 is continuous with the first distal portion 112 of the outer tube 110, which refers to that at least a portion of the first tube body 111 and at least a portion of the first distal portion 112 of the outer tube 110, at the sides close to each other, are in contact and connected (e.g., the two portions are butted end to end) with each other, so that the first tube body 111 and the first distal portion 112 are continuous and uninterrupted in space. For example, the first tube body 111 and the first distal portion 112 are integrally formed. Of course, this is only exemplary and does not constitute a limitation of the embodiments of the present disclosure. As long as the first tube body 111 and the first distal portion 112 can be two separate portions which are directly connected, no exhaustive enumeration or further elaboration is provided herein. It should be noted that the interpretation of "continuous" in other embodiments of the present disclosure can also refer to the description here, which is not particularly explained.

For example, as shown in Figs. 6 and 7, the nozzle tube 120 is arranged to extend along the first direction (i.e., the nozzle tube 120 extends along the first direction as a whole, no bent outlet is provided at the distal end of the nozzle tube 120, and the central axis of the entire nozzle tube 120 is along the first direction). The distal outlet end face of the nozzle tube 120 (for clarity and conciseness, it can be denoted as the second distal outlet end face 122a) is obliquely arranged relative to the first direction (the first direction intersects with the second distal outlet end face 122a but is not perpendicular thereto), i.e., the second distal outlet end face 122a is in a beveled outlet shape relative to the first direction.

For example, in the example of Fig. 6, the second direction corresponding to the jet flow direction is perpendicular to the first distal outlet end face 112a of the outer tube 110.

In some examples, the beveled outlet shape satisfying the corresponding requirements of the first distal outlet end face 112a of the outer tube 110 can be achieved by means of oblique cutting, and the beveled outlet shape satisfying the corresponding requirements of the second distal outlet end face 122a of the nozzle tube 120 can also be achieved by means of oblique cutting. For example, the orifice in the distal end of the nozzle tube 120 is a straight orifice. For example, an end plane where the straight orifice is located can be fixed on the beveled outlet of the nozzle tube 120. This is only exemplary and does not constitute a limitation of the embodiments of the present disclosure.

By combining the beveled outlet of the outer tube and the beveled outlet of the nozzle tube, the embodiments of the present disclosure enable the jet flow direction to deflect by a certain angle relative to the axial direction of the outer tube and enable the outlet to fit the tissue surface, which not only satisfies clinical functional requirements but also achieves better compatibility with a 5 mm trocar (e.g., the design with beveled outlet of the outer tube 110 has no adverse effect when passing through the 5 mm trocar), better machinability (e.g., the processing of the outer tube and the nozzle tube is simple) as well as better assemblability (e.g., the nozzle tube has fewer axial and circumferential constraints when moving inside the outer tube, which enables the assembly process to be relatively simple).

For example, as shown in Fig. 6, a plane where the first distal outlet end face 112a of the outer tube 110 is located is parallel to a plane where the second distal outlet end face 122a of the nozzle tube 120 is located, i.e., the beveled outlet shape of the first distal outlet end face 112a of the outer tube 110 has the same slope and inclination direction as those of the beveled outlet shape of the second distal outlet end face 122a of the nozzle tube 120. In this way, the embodiments of the present disclosure can further enhance the advantages in terms of clinical functional requirements, compatibility, machinability, and assemblability. Here, the slope refers to an angular coefficient of the acute angle formed between the first distal outlet end face 112a or the second distal outlet end face 122a and the first direction, i.e., the inclination degree of the first distal outlet end face 112a or the second distal outlet end face 122a relative to the first direction.

For example, as shown in Figs. 8 and 9, the first tube body 111 of the outer tube 110 is continuous and coaxial with the first distal portion 112 of the outer tube 110 (i.e., the outer tube 110 extends along the first direction as a whole, no curved outlet is provided at the distal end of the outer tube 110, and the central axis of the entire outer tube 110 is along the first direction). The first distal outlet end face 112a of the outer tube 110 is obliquely arranged relative to the first direction, i.e., the first distal outlet end face 112a of the outer tube 110 is in a beveled outlet shape relative to the first direction.

For example, as shown in Figs. 8 and 9, the nozzle tube 120 is arranged to extend along the first direction (i.e., the nozzle tube 120 extends along the first direction as a whole, no bent outlet is provided at the distal end of the nozzle tube 120, and the central axis of the entire nozzle tube 120 is along the first direction). The distal outlet end face of the nozzle tube 120 (which is an end face at the outlet of the distal end of the nozzle tube 120, and may be denoted as the second distal outlet end face 122a for clarity and conciseness) is perpendicular to the first direction, i.e., the second distal outlet end face 122a is in a straight outlet shape which is perpendicular to the first direction. The orifice is configured in an oblique orifice shape relative to the second distal outlet end face 122a, so that the medical liquid is ejected along the second direction B. The oblique orifice shape in some embodiments of the present disclosure refers to that the axis line of the hole (i.e., the orifice) is not parallel but oblique to the first direction of the nozzle tube 120. In other words, the axis line of the orifice is not perpendicular to the second distal outlet end face 122a.

By combining the beveled outlet of the outer tube and the oblique orifice of the inner tube, the embodiments of the present disclosure enable the jet flow direction to deflect by a certain angle relative to the axial direction of the outer tube and enable the outlet to fit the tissue surface, which not only satisfies clinical functional requirements but also achieves better compatibility with a 5 mm trocar (e.g., the design with beveled outlet of the outer tube 110 has no adverse effect when passing through the 5 mm trocar), better machinability (e.g., the processing of the outer tube and the nozzle tube is simple), and better assemblability (e.g., the nozzle tube has fewer axial and circumferential constraints when moving inside the outer tube, which enables the assembly process to be relatively simple).

In some embodiments of the present disclosure, the orifice in an oblique orifice shape is formed by means of drilling an oblique orifice in the straight-shaped end face of the outlet (the vertical end face as shown in Fig. 8, for example, the cross-section of the vertical end face is a perfect circle) of the nozzle tube 120 extending along the first direction. In this way, since the jet flow direction is consistent with the oblique orifice direction of the nozzle tube, the jet flow direction (i.e., the second direction) is deflected relative to the central axes of the nozzle tube and the outer tube extending along the first direction. Therefore, the embodiments of the present disclosure allow for simple operations during the processing and fixing processes.

For example, in the example of Fig. 8, the second direction corresponding to the jet flow direction is perpendicular to the first distal outlet end face 112a of the outer tube 110.

In some examples, the acute angle formed between the second direction and the first direction is 10° to 50°. Thus, the embodiments of the present disclosure can well satisfy the clinical functional requirements and the structural movement requirements for the device in an operating state. It should be noted that this is only exemplary and does not constitute a limitation of the embodiments of the present disclosure, which can be freely adjusted according to actual application needs and is not exhaustively described or particularly explained here.

In some examples, the proximal portion of the nozzle tube 120 is configured to be fixed relative to the outer tube 110, and the driving device 130 is configured to drive the orifice in the distal portion of the nozzle tube 120 (which may be denoted as the second distal portion of the nozzle tube 120, e.g., the second distal portion 122 of the nozzle tube 120 shown in Fig. 4) to perform, relative to the outer tube 110, a one-dimensional reciprocating swing (also referred to as lateral swing) along the radial direction of the outer tube 110 or a two-dimensional circular rotation (also referred to as orbital rotation) around the direction of the central axis of the outer tube 110.

In the medical water jet device according to the embodiments of the present disclosure, by arranging the outlet of the outer tube to be oblique to but not perpendicular to the axis line of the main body of the outer tube under the condition of lateral swing or orbital rotation, and by cleverly designing the outer tube and/or the inner tube to enable the jet flow direction to form an oblique angle with relative to the axis line of the main body of the outer tube, the situation that the deflection direction of the jet flow changes periodically due to the rotation of the water jet nozzle on its own axis cannot occur. For example, since the position of the water jet nozzle in the embodiments of the present disclosure only undergoes lateral swing or orbital rotation without autorotation, the embodiments of the present disclosure can satisfy the requirement of the operator that the jet flow of the nozzle tube deflects by a predictable fixed angle, which well satisfies the clinical functional requirements and the structural requirements of the system for the device.

At least one embodiment of the present disclosure further provides a medical water jet system, including a liquid supply unit, a pressure pump, and a medical water jet device. The liquid supply unit is configured to provide medical liquid. The pressure pump is configured to apply a pressure to the medical liquid to input the medical liquid into the nozzle tube. The medical water jet device of the medical water jet system may be the medical water jet device of any one of the above embodiments, which is not particularly explained here. For the technical effects of the medical water jet system of the embodiments of the present disclosure, reference can be made to the description of the medical water jet device above, which is not particularly explained here.

The following points need to be explained:
(1) The drawings of the embodiments of the present disclosure only involve the structures related to the embodiments of the present disclosure, and other structures can refer to the usual designs.
(2) Without conflict, the embodiments of the present disclosure and the features in the embodiments can be combined with each other to obtain new embodiments.

The above merely refers to specific implementations of the present disclosure, but the scope of protection of the present disclosure is not limited thereto. The scope of protection of the present disclosure shall be subject to the scope of protection of the appended claims.

## Claims

1. A medical water jet device, comprising:
an outer tube, comprising a first tube body extending along a first direction and a first distal portion having a first distal outlet end face, wherein a plane defined by the first distal outlet end face is obliquely arranged relative to the first direction;
a nozzle tube, extending through the outer tube and configured to input medical liquid at a proximal end, wherein a gap space is provided between an outer wall of the nozzle tube and an inner wall of the outer tube, an orifice configured to output the medical liquid is provided at a distal end of the nozzle tube, and the medical liquid is ejected along a second direction that is oblique to the first direction; and
a driving device, connected to at least a portion of the nozzle tube and configured to drive the nozzle tube to move.

2. The medical water jet device according to claim 1, wherein
the first distal portion of the outer tube is in a curved shape; and
the nozzle tube comprises a second tube body extending along the first direction and a second distal portion in a curved shape.

3. The medical water jet device according to claim 2, wherein
the first distal outlet end face is perpendicular to a central axis of the first distal portion, and a second distal outlet end face on the second distal portion is perpendicular to a central axis of the second distal portion.

4. The medical water jet device according to claim 2, wherein
a central axis of the first distal portion is parallel to a central axis of the second distal portion.

5. The medical water jet device according to claim 1, wherein
the first tube body of the outer tube is continuous and coaxial with the first distal portion, and the first distal outlet end face of the outer tube is obliquely arranged relative to the first direction; and
the nozzle tube is arranged to extend along the first direction, and a second distal outlet end face of the nozzle tube is obliquely arranged relative to the first direction.

6. The medical water jet device according to claim 5, wherein
the plane defined by the first distal outlet end face is parallel to a plane defined by the second distal outlet end face.

7. The medical water jet device according to claim 1, wherein
the first tube body of the outer tube is continuous and coaxial with the first distal portion, and the first distal outlet end face of the outer tube is obliquely arranged relative to the first direction; and
the nozzle tube is arranged to extend along the first direction, a second distal outlet end face of the nozzle tube is perpendicular to the first direction, and the orifice is configured in an oblique orifice shape relative to the second distal outlet end face so that the medical liquid is ejected along the second direction.

8. The medical water jet device according to any one of claims 1-7, wherein
the second direction is perpendicular to the first distal outlet end face.

9. The medical water jet device according to any one of claims 1-7, wherein
an acute angle formed between the second direction and the first direction is 10° to 50°.

10. The medical water jet device according to any one of claims 1-7, wherein
a proximal portion of the nozzle tube is configured to be fixed relative to the outer tube, and the driving device is configured to drive the nozzle tube such that the orifice of the nozzle tube performs, relative to the outer tube, a one-dimensional reciprocating swing along a radial direction of the outer tube or a two-dimensional circular rotation around a direction of a central axis of the outer tube.

11. A medical water jet system, comprising:
the medical water jet device according to any one of claims 1-10;
a liquid supply unit, configured to provide the medical liquid; and
a pressure pump, configured to apply a pressure to the medical liquid to input the medical liquid into the nozzle tube.
